# EUROPEAN PATENT APPLICATION

(11) **EP 4 613 847 A1**
(43) Date of publication of application: **10.09.2025**
(21) Application number: 23886015.9
(22) Date of filing: 21.09.2023
(51) Int. Cl.: C12N 5/04, A61K 8/9789, A61Q 19/00, C12Q 1/24

(54) **METHOD FOR ISOLATING PLANT-DERIVED EXOSOME, EXOSOME ISOLATED THEREBY, AND COSMETIC USE THEREOF**

(30) Priority: 04.11.2022 KR 20220145732
(71) Applicant: Activon Co., Ltd., Cheongju-si, Chungcheongbuk-do 28104 (KR); THE, DABOM CORPORATION, Seoul 03722 (KR)
(72) Inventor: JEON, Ju Hee, Cheongju-si Chungcheongbuk-do 28113 (KR); KIM, Yoon Ah, Cheongju-si Chungcheongbuk-do 28781 (KR); CHO, Se Hee, Seoul 07691 (KR); JUNG, Sung Won, Yongin-si Gyeonggi-do 16909 (KR); CHO, Youn Ki, Yongin-si Gyeonggi-do 17005 (KR); JUNG, Hyo Il, Seoul 06515 (KR); PARK, Sun Young, Seoul 03726 (KR)
(74) Representative: Vossius & Partner Patentanwälte Rechtsanwälte mbB
(86) International application number: PCT/KR2023/014371
(87) International publication number: WO 2024/096318

(57) **Abstract**

The present disclosure provides an exosome isolation method that can increase exosome isolation efficiency, and a cosmetic composition containing exosomes with improved anti-wrinkle, cell proliferation, wound healing, or whitening effects. The present disclosure provides a method for extracting plant-derived exosomes, the method includes (a) washing and juicing a plant to obtain a plant juice; (b) firstly centrifuging the plant juice to obtain a first supernatant; (c) secondly centrifuging the first supernatant to obtain a second supernatant; (d) subjecting the second supernatant to tangential flow filtration (TFF) to obtain an isolated substance; and (e) adding antibody-attached beads to the isolated substance to obtain an extract containing plant-derived exosomes. Another embodiment of the present disclosure provides a cosmetic composition including 0.1 to 20 wt% of plant-derived exosomes based on the total weight of the composition.

## Description

### TECHNICAL FIELD

### Cross-references to related applications.

This application claims the benefit of priority based on Korean Patent Application No. 10-2022-0145732 filed on November 4, 2022, and the entire contents of the Korean patent application are incorporated herein by reference.

The present disclosure relates to a method for extracting plant-derived exosome, exosome isolated thereby, and cosmetic use thereof.

### BACKGROUND ART

Exosomes refer to small vesicles with a membrane structure secreted by various cells and is defined as a type of extracellular vesicles (EVs). All cells secrete EVs to exchange information with other cells or the external environment. Exosomes range in size from approximately 50 to 200 nm and contain bioactive substances such as proteins, lipids, and nucleic acids. Exosomes exist in various cells, including those of mammals, bacteria, and plants, and reflect the state of their originating cells, so they can be used for diagnosis and treatment. Exosomes are bilayer phospholipid structures that easily penetrate cells and perform diverse physiological and pathological functions, such as immune responses and signal transduction.

Recent studies have explored the various benefits of plant-derived exosomes, revealing effects such as antioxidation and anti-inflammation. These plant-derived exosomes are natural nanoparticles that contain bioactive and signaling substances secreted by plant cells, facilitating intercellular movement and absorption, and the plant-derived exosomes are known to exhibit no toxicity compared to exosomes derived from mammals.

Due to their diverse advantages and activities, these exosomes are considered a promising material for applications in pharmaceuticals, cosmetics, and food. However, their structural characteristic as bilayer phospholipid membranes poses challenges, such as low dispersibility and a tendency to aggregate. In addition, exosomes are also unstable at high temperatures and prone to damage during the manufacturing process of cosmetic formulations, and these properties can reduce the stability of exosomes in formulations and cause precipitation. Therefore, improving the dispersibility of exosomes in aqueous solutions is necessary to increase their stability in formulations and maintain their continuous activity.

To date, in most studies, exosomes have been isolated through a ultracentrifugation method based on differences in density and size from the fluid phase. However, the ultracentrifugation method has disadvantages such as the risk of breaking exosomes due to excessive shock, being labor-intensive, and time-consuming. In addition, the ultracentrifugation method has low exosome yield and cannot separate a large amount of samples at once, which lowers its industrial applicability.

Another exosome isolation method is a precipitation method, where water-excluding polymers such as polyethylene glycol (PEG) bind water molecules and extract less soluble components from the solution. The precipitation method with a PEG-containing precipitation solution is easy to use and does not require special equipment, but it has the disadvantages of difficult mass production and exosome aggregation.

Meanwhile, Meanwhile, Glycyrrhiza uralensis, a perennial plant in the Fabaceae in the order Rosales, has long been used as a medicinal plant. Glycyrrhiza uralensis is effective for detoxification, hepatitis, urticaria, dermatitis, eczema, and has antitussive, expectorant, muscle relaxant, diuretic, and anti-inflammatory properties, as well as the ability to suppress peptic ulcers.

### [Prior Art Documents]

### [Patent Documents]

1. Korean Patent Registration No. 10-2125567 (June 22, 2020)

### DISCLOSURE OF THE INVENTION

### TECHNICAL PROBLEM

The present disclosure is to provide a method for isolating exosomes with enhanced exosome isolation efficiency.

The present disclosure improves the efficiency of exosome isolation by further isolating exosomes from extracellular vesicles using antibody-attached beads.

In addition, the present disclosure is to provide a cosmetic composition containing exosomes with improved anti-wrinkle, cell proliferation, wound healing, or whitening effects.

### TECHNICAL SOLUTION

To achieve the above object, one embodiment of the present disclosure provides a method for extracting plant-derived exosomes, the method comprising: (a) washing and juicing a plant to obtain a plant juice; (b) firstly centrifuging the plant juice to obtain a first supernatant; (c) secondly centrifuging the first supernatant to obtain a second supernatant; (d) subjecting the second supernatant to tangential flow filtration (TFF) to obtain an isolated substance; and (e) adding antibody-attached beads to the isolated substance to obtain an extract containing plant-derived exosomes.

In the present disclosure, the plant in step (a) may be Glycyrrhiza uralensis.

In the present disclosure, the first centrifugation in step (b) is performed at 1,000 xg to 3,000 xg for 10 to 50 minutes.

In the present disclosure, the second centrifugation in step (b) is performed at 8,000 xg to 12,000 xg for 40 to 80 minutes.

In the present disclosure, the tangential flow filtration in step (d) is performed using a TFF filter with a molecular weight cutoff (MWCO) of 50,000 Da to 200,000 Da.

In the present disclosure, the antibody in step (e) is a plant-derived tetraspanin antibody.

Another embodiment of the present disclosure provides a plant-derived exosome extracted by the method described above.

Yet another embodiment of the present disclosure provides a cosmetic composition comprising 0.1 to 20 wt% of the plant-derived exosome based on the total weight of the composition.

In the present disclosure, the cosmetic composition is for anti-wrinkle, cell proliferation, wound healing, or whitening.

### ADVANTAGEOUS EFFECTS

The method for isolating plant-derived exosomes according to the present disclosure can improve the isolation efficiency of exosomes by using tangential flow filtration and antibody-attached beads together.

In addition, the cosmetic composition containing the plant-derived exosomes according to the present disclosure can exhibit excellent anti-wrinkle, cell proliferation, wound healing, or whitening.

### BRIEF DESCRIPTION OF THE DRAWINGS

FIG. 1 is a diagram illustrating the results of NTA analysis and TEM analysis of Glycyrrhiza uralensis-derived exosomes of the Embodiment.
FIG. 2 is graphs illustrating the results of NTA analysis to confirm the size distribution and number of particles of exosomes of the Embodiment and Comparative Examples 1 and 2.
FIG. 3 is graphs illustrating the results of NTA analysis to confirm the size distribution and number of particles of exosomes of the Embodiment and Comparative Example 3.
FIG. 4 is graphs illustrating the evaluation result of the cell proliferation ability of exosomes of the Embodiment and Comparative Example 3.
FIG. 5 is a graph illustrating the evaluation result of the whitening effects of exosomes of the Embodiment and Comparative Example 3.
FIG. 6 is a graph illustrating the evaluation result of the procollagen type I production ability of exosomes of the Embodiment and Comparative Example 3.
FIG. 7 is a view illustrating the evaluation result of the skin cell wound healing ability of exosomes of the Embodiment.

### BEST MODE FOR CARRYING OUT THE INVENTION

Hereinafter, the embodiment of the present disclosure will be described in detail with reference to the accompanying drawings so that those skilled in the art can easily implement the present disclosure. However, the present disclosure can be implemented in various different forms and is not limited to the embodiments described herein.

The term "exosome" as used in the present disclosure collectively refers to exosomes or exosome-like extracellular vesicles having a size of about 50 to 200 nm extracted from plants, preferably Glycyrrhiza uralensis.

The present disclosure provides a method for isolating plant-derived exosomes, the method comprising: (a) washing and juicing a plant to obtain a plant juice; (b) firstly centrifuging the plant juice to obtain a first supernatant; (c) secondly centrifuging the first supernatant to obtain a second supernatant; (d) subjecting the second supernatant to tangential flow filtration (TFF) to obtain an isolated substance; and (e) adding antibody-attached beads to the isolated substance to obtain an extract containing plant-derived exosomes.

In the present disclosure, the plant in step (a) may be Centella asiatica, and specifically fresh or dried Glycyrrhiza uralensis, with fresh plants being most preferred. In addition, washing may be performed using ethanol or distilled water, and plant juicing may be performed using a juicer commonly used in the art, and preferably a screw juicer may be used. The stirring speed of the screw juicer is preferably 20 to 50 rpm.

The centrifugation method of steps (b) and (c) is an isolation method using the density difference between a solid and a liquid surrounding the solid, and means a method of accelerating the sedimentation of the solid using centrifugal force. Centrifugation has the advantages of being able to continuously repeat the process, being able to process a large amount in a short time, and being easy to operate in a sterile state.

The first centrifugation in step (b) is performed to obtain a first supernatant by low-speed centrifugation of the plant juice, and may be performed at 1,000 xg to 3,000 xg for 10 to 50 minutes, preferably at 1,500 xg to 2,500 xg for 20 to 40 minutes. If it is out of the above range, the isolation efficiency of exosomes may be lowered, so the above range is preferable.

The second centrifugation in step (c) is performed to obtain a second supernatant by high-speed centrifugation of the first supernatant, and may be performed at 8,000xg to 12,000 xg for 40 to 80 minutes, preferably at 9,000 xg to 11,000 xg for 50 to 70 minutes. If it is out of the above range, the isolation efficiency of exosomes may be lowered, so the above range is preferable.

In addition, in the present disclosure, after step (c), in order to improve exosome isolation efficiency, the obtained second supernatant may be removed from the residue with a 0.2 to 0.3 µm filter under reduced pressure conditions.

The tangential flow filtration (TFF) in step (d) is a filtration method in which a solution flows in a direction perpendicular to the filtration membrane, filtering out small-sized impurities present in the solution and isolating large-sized exosomes.

The rapid flow of the feed solution in TFF acts to reduce concentration polarization (product concentration at the pore surface) by 'sweeping' the membrane or hollow fiber surface. In addition, the TFF method prevents the buildup of contaminants that may clog pores. This rapid cross flow reduces pressure, which may force some of the feed solution and dissolved molecules smaller than the pores of the membrane or hollow fiber to pass through the filter. The solution that has passed through the pores is called filtrate or permeate, and molecules or particles larger than the pores remain in the feed solution and are effectively concentrated. In a preferred embodiment, the TFF of the present disclosure is an ultrafiltration system, and as a result of performing the TFF method as described above, the centrifuged extract may be efficiently concentrated to a volume of 1/10 to 1/100. Ultrafiltration is located in the intermediate region between microfiltration and reverse osmosis, and is a method of isolating specific substances by the size difference between membrane pores and solutes. An ultrafiltration membrane may exhibit its isolation performance as a molecular weight cutoff (MWCO), which is defined as the minimum molecular weight of a solute that exhibits a rejection of 90% or more by the membrane.

In step (d), tangential flow filtration may be performed using a TFF filter with a molecular weight cutoff (MWCO) of 50,000 Da to 200,000 Da, preferably a TFF filter with a molecular weight cutoff of 80,000 Da to 120,000 Da.

Step (e) is a step of obtaining an extract containing exosomes from isolated substance containing exosomes isolated by tangential flow filtration, which may be performed by adding antibody-attached beads, and the antibody-attached beads may be manufactured by mixing antibodies and beads in a buffer.

In the present disclosure, the antibody binds to exosomes or exosome-like extracellular vesicles in the isolated substance to enable final isolation of exosomes, and may be an antibody of a protein present in the plant extracellular vesicle membrane, and to improve the exosome extraction efficiency, it may be preferably tetraspanin, a membrane protein of plant extracellular vesicles, and more preferably tetraspanin-8 (TET8), and the beads may be used without limitation as long as they are generally used as a support for antibody attachment in the art.

The tetraspanin-8 used in the present disclosure is structurally similar to mammalian CD63 and is a closely related gene abundantly present in plants. Tetraspanin-8 may serve as a specific marker for plant exosomes.

According to the present disclosure, as a result of attempting to isolate Glycyrrhiza uralensis-derived exosomes using anti-tetraspanin antibodies bound to a support such as beads, it was confirmed that the isolation efficiency increased and the yield improved.

In the present disclosure, the amount of antibody-attached beads used may be 50 to 150 parts by weight based on 100 parts by weight of the isolated substance containing exosomes, preferably 80 to 120 parts by weight, and most preferably 100 parts by weight. If the amount of antibody-attached beads used is less than 50 parts by weight, the isolation efficiency of exosomes may be lowered, and if it exceeds 150 parts by weight, the exosome isolation efficiency does not increase significantly compared to the added amount, which is not economical.

In the present disclosure, after step (e), a step of finally isolating exosomes from the extract containing exosomes may be further included.

The plant-derived exosomes separated according to the present invention may have a size of 50 to 200 nm, preferably 100 to 200 nm, and may contain 100×10¹⁰ to 1,000×10¹⁰ particles per 1 ml of extract, preferably 200×10¹⁰ to 500×10¹⁰ particles per 1 ml of extract.

Another embodiment of the present disclosure is a plant-derived exosome isolated by the isolation method described above, and the characteristics thereof are the same as described above, and thus will not be described below.

In addition, yet another embodiment of the present disclosure is a cosmetic composition containing 0.1 to 20 wt% of the isolated plant-derived exosomes based on the total weight, and the cosmetic composition may be for anti-wrinkle, cell proliferation, wound healing, or whitening.

The anti-wrinkle effect of the present disclosure was confirmed through procollagen type I production ability, the cell proliferation effect was confirmed through HaCaT and Hs68 cells, the wound healing effect was confirmed through the wound healing ability of skin cells, and the whitening effect was confirmed through the amount of melanin production.

The cosmetic composition of the present disclosure may include commonly permitted ingredients without limitation in addition to plant-derived exosomes, such as conventional adjuvants such as antioxidants, stabilizers, solubilizers, vitamins, pigments and fragrances, as well as carriers.

The cosmetic composition according to this disclosure may be manufactured into one or more formulations selected from the group consisting of solutions, topical ointments, creams, foams, nutrient toner, softening toner, packs, softener, emulsion, makeup bases, essences, soaps, liquid cleansers, bath preparations, sunscreen creams, sunscreen oils, suspensions, emulsions, pastes, gels, lotions, powders, soaps, surfactant-containing cleansers, oils, powder foundations, emulsion foundations, wax foundations, patches, and sprays, but is not limited thereto.

In addition, the cosmetic composition in the present disclosure may also include one or more cosmetically acceptable carriers commonly used in general skin cosmetics, such as serum, water, surfactants, moisturizers, lower alcohols, thickeners, chelating agents, pigments, preservatives, or fragrances, but is not limited thereto. The cosmetically acceptable carriers included in the cosmetic composition of the present disclosure may vary depending on the formulation.

If the formulation of the present disclosure is an ointment, paste, cream, or gel, carrier ingredients such as animal oils, vegetable oils, waxes, paraffin, starch, tragacanth, cellulose derivatives, polyethylene glycol, silicone, bentonite, silica, talc, zinc oxide, or mixtures thereof may be used.

If the formulation of the present disclosure is a powder or spray, carrier ingredients such as lactose, talc, silica, aluminum hydroxide, calcium silicate, polyamide powder, or mixtures thereof may be used, and particularly in the case of a spray, propellants such as chlorofluorohydrocarbons, propane/butane, or dimethyl ether may additionally be included.

If the formulation of the present disclosure is a solution or emulsion, carrier ingredients such as solvents, solubilizers, or emulsifiers may be used, including water, ethanol, isopropanol, ethyl carbonate, ethyl acetate, benzyl alcohol, benzyl benzoate, propylene glycol, or 1,3-butylene glycol oils, particularly cottonseed oil, peanut oil, corn germ oil, olive oil, castor oil, sesame oil, glycerol aliphatic esters, polyethylene glycol, or fatty acid esters of sorbitan.

If the formulation is a suspension, liquid diluents such as water, ethanol, or propylene glycol, suspending agents such as ethoxylated isostearyl alcohol, polyoxyethylene sorbitol esters, or polyoxyethylene sorbitan esters, and materials like microcrystalline cellulose, aluminum metahydroxide, bentonite, agar, or tragacanth may be used as carrier ingredients

If the formulation is soap, carrier ingredients such as alkali metal salts of fatty acids, hemiester salts of fatty acids, hydrolyzed protein of fatty acids, isethionate, lanolin derivatives, aliphatic alcohols, vegetable oils, glycerol, or saccharides may be used.

If the formulation is a surfactant-containing cleansing product, carrier ingredients such as alkyl sulfate, alkyl alcohol ether sulfate, sulfosuccinate monoesters, isethionate, imidazolinium derivatives, methyltaurate, sarcosinate, fatty acid amide ether sulfate, alkylamidobetaine, aliphatic alcohols, fatty acid glycerides, fatty acid diethanolamides, vegetable oils, lanolin derivatives, or ethoxylated glycerol fatty acid esters may be used.

Hereinafter, the present disclosure will be described in more detail through specific embodiments. However, the following embodiments are only intended to illustrate the present disclosure, and the contents of the present disclosure are not limited to the following examples.

### Embodiment

1 kg of fresh Glycyrrhiza uralensis was washed with ethanol (90%) and distilled water, and then the remaining moisture was removed. The washed Glycyrrhiza uralensis was juiced using a general juicer at a low speed of 30 rpm with a screw juicer, and the obtained Glycyrrhiza uralensis juice was filtered through a mesh to remove suspended solids.

The Glycyrrhiza uralensis juice was subjected to low-speed centrifugation (2,000 xg, 30 minutes, 4°C) to remove the residue, and the first supernatant was collected, and the first supernatant was subjected to high-speed centrifugation (12,000 xg, 60 minutes, 4°C) to remove the remaining residue, and the second supernatant was collected.

The second supernatant was filtered through a 0.22 µm filter under reduced pressure conditions to finally remove the residue.

Exosomes were extracted from the second supernatant from which the residue was removed through a tangential-flow filtration (TFF) system. Specifically, exosomes were isolated and purified using a tangential flow filtration system equipped with a 100,000 Da MWCO membrane.

After attaching TET8 antibody to beads by mixing TET8 antibody (TET8 antibody, Phytoab) and beads (7um streptavidin coated beads, Spherotech) in a buffer, add 500 µl of TET8 antibody attached beads to 500 µl of the isolated substance isolated using TFF and process for 10 minutes using a HOMM chip to obtain an extract containing exosomes. Subsequently, extracellular vesicles (EVs) were detached using Elution buffer (The DABOM) and then exosomes were finally isolated.

### Comparative Example 1

1 kg of fresh Glycyrrhiza uralensis was washed with ethanol (90%) and distilled water, and then the remaining moisture was removed. The washed Glycyrrhiza uralensis was juiced using a general juicer at a low speed of 30 rpm with a screw juicer, and the obtained Glycyrrhiza uralensis juice was filtered through a mesh to remove suspended solids.

The Glycyrrhiza uralensis juice was subjected to low-speed centrifugation (2,000 xg, 30 minutes, 4°C) to remove the residue, and the first supernatant was collected, and the first supernatant was subjected to high-speed centrifugation (12,000 xg, 60 minutes, 4°C) to remove the remaining residue, and the second supernatant was collected.

The second supernatant was filtered through a 0.22 µm filter under reduced pressure conditions to finally remove the residue.

The second supernatant from which the residue was removed was subjected to ultracentrifugation (150,000 xg, 70 min, 4°C) to obtain a third supernatant and finally extract exosomes.

### Comparative Example 2

1 kg of fresh Glycyrrhiza uralensis was washed with ethanol (90%) and distilled water, and then the remaining moisture was removed. The washed Glycyrrhiza uralensis was juiced using a general juicer at a low speed of 30 rpm with a screw juicer, and the obtained Glycyrrhiza uralensis juice was filtered through a mesh to remove suspended solids.

The Glycyrrhiza uralensis juice was subjected to low-speed centrifugation (2,000 xg, 30 minutes, 4°C) to remove the residue, and the first supernatant was collected, and the first supernatant was subjected to high-speed centrifugation (12,000 xg, 60 minutes, 4°C) to remove the remaining residue, and the second supernatant was collected.

The second supernatant was filtered through a 0.22 µm filter under reduced pressure conditions to finally remove the residue.

After mixing the second supernatant with the PEG/Dextran solution, centrifugation was performed at 1,000 xg for 10 minutes at 4°C. After centrifugation, the supernatant was removed to extract exosomes.

### Comparative Example 3

Glycyrrhiza uralensis extract was purchased from a commercial source (World Costec) and prepared. The Glycyrrhiza uralensis extract was subjected to low-speed centrifugation (2,000 xg, 30 minutes, 4°C) to remove residues, and the first supernatant was collected. The first supernatant was then subjected to high-speed centrifugation (12,000 xg, 60 minutes, 4°C) to remove remaining residues, and the second supernatant was collected.

The second supernatant was filtered through a 0.22 µm filter under reduced pressure conditions to finally remove the residue and extract exosomes.

Exosomes were extracted from the second supernatant from which the residue was removed through a tangential-flow filtration (TFF) system. Specifically, exosomes were isolated and purified using a tangential flow filtration system equipped with a 100,000 Da MWCO membrane to extract exosomes.

### Experimental Example 1

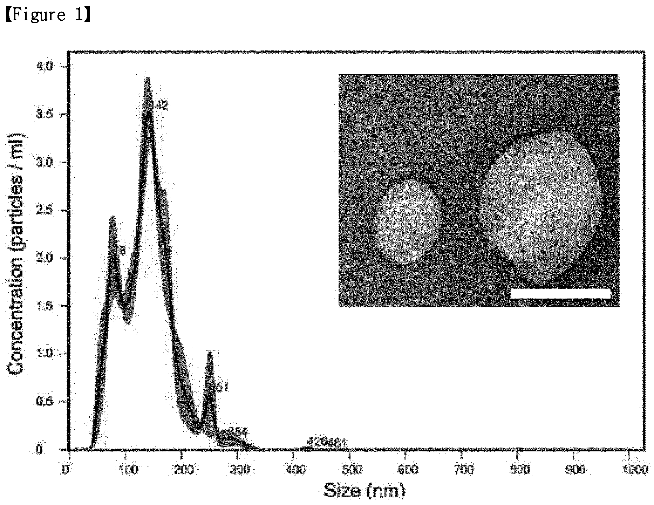

In order to confirm the particle size distribution, the number of particles per unit volume, and the shape of the exosomes extracted in the embodiment, they were analyzed by Nanoparticle Tracking Analysis (NTA) and Transmission Electron Microscope (TEM), and the results are shown in Fig. 1.

Referring to FIG. 1, it may be confirmed that the size of the exosome particles extracted according to the Embodiment averaged 100 to 200 nm, and the number per unit volume of 1 ml was found to be about 320 x 10¹⁰. In addition, as a result of TEM analysis, the presence of spherical particles with a lipid bilayer structure, approximately 150 nm in size was confirmed.

### Experimental Example 2

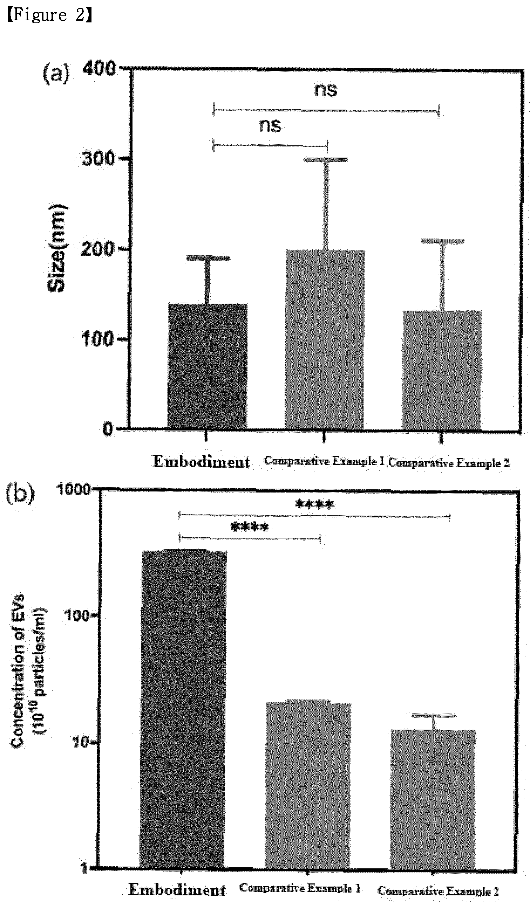

In order to confirm the particle size distribution and the number of particles per unit volume of the exosomes extracted in the Embodiment and Comparative Examples 1 and 2, they were analyzed by Nanoparticle Tracking Analysis (NTA), and the results are shown in FIG. 2.

FIG. 2A is a diagram illustrating the size distribution of exosomes derived from Glycyrrhiza uralensis, and FIG. 2B is a diagram illustrating the number of particles per 1 ml of exosomes.

Referring to FIG. 2A, it was confirmed that the size of the exosome particles of the Embodiment was an average of 100 to 200 nm, the size of the exosome particles of Comparative Example 1 was an average of 200 to 300 nm, and the size of the exosome particles of Comparative Example 2 was an average of 100 to 200 nm.

Referring to FIG. 2B, the number per unit volume of 1 ml is about 320 x 10¹⁰ in the case of the Embodiment, about 30 x 10¹⁰ in Comparative Example 1, and about 20 x 10¹⁰ in Comparative Example 2. The results confirm that the largest amount of exosomes could be extracted when TFF and antibody-attached beads were used (Embodiment).

### Experimental Example 3

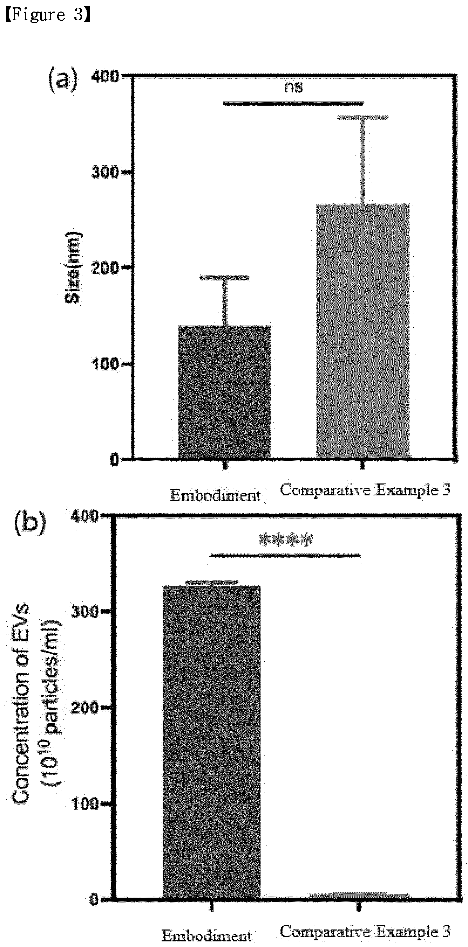

In order to confirm the particle size distribution and the number of particles per unit volume of the exosomes extracted in the Embodiment and Comparative Example 3, they were analyzed by Nanoparticle Tracking Analysis (NTA), and the results are shown in FIG. 3.

FIG. 3A is a diagram illustrating the size distribution of exosomes derived from Glycyrrhiza uralensis, and FIG. 3B is a diagram illustrating the number of particles per 1 ml of exosomes.

Referring to FIG. 3A, it was confirmed that the size of the exosome particles of the Embodiment was an average of 100 to 200 nm, and the size of the exosome particles of Comparative Example 3 was an average of 200 to 300 nm.

Referring to FIG. 3B, the number per unit volume of 1 ml is about 320 x 10¹⁰ in the case of the Embodiment, and about 1 x 10¹⁰ in Comparative Example 3. The results confirm that a much larger amount of exosomes could be extracted from the Glycyrrhiza uralensis extract when using TFF and antibody-attached beads (Embodiment) than when using TFF only (Comparative Example 3).

### Experimental Example 4

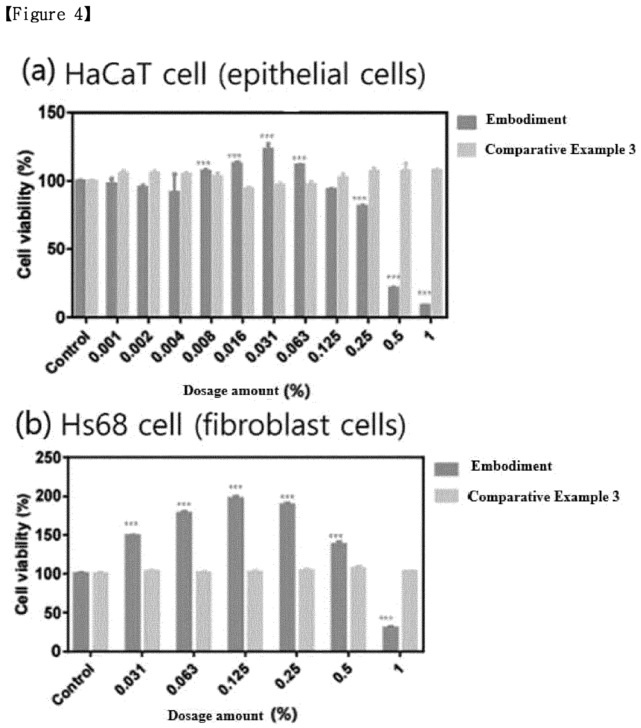

In order to measure the cell proliferation effect of the exosomes of the Example and Comparative Example 3, the following experiment was conducted using human epidermal cells (HaCaT) and human dermal fibroblasts (Hs68), and the results are shown in FIG. 4.

HaCaT and Hs68 cells were seeded at 1 × 10³ cells/100 µl per well in a 96-well plate and cultured for 24 hours. The negative control (Control; untreated group), Embodiment, and Comparative Example 3 exosomes (2 × 10¹⁰ particles/ml (100% basis)) were treated at concentrations of 0.001%, 0.002%, 0.004%, 0.008%, 0.016%, 0.031%, 0.063%, 0.125%, 0.25%, 0.5%, and 1%.

After 24 hours of treatment, the absorbance was measured at 450 nm using CCK8 (Dojindo, CK04-13) reagent to assess changes in cell viability.

FIG. 4A is a graph illustrating the survival rate of human epidermal cells, and FIG. 4B is a graph illustrating the survival rate of human dermal fibroblasts.

Referring to FIG. 4A, in the case of Glycyrrhiza uralensis-derived exosomes (Embodiment), it may be confirmed that it shows superior cell proliferation effect than Glycyrrhiza uralensis extract-derived exosomes (Comparative Example 3) from 0.008% to 0.063%, and shows the most excellent effect at 0.031%.

Referring to FIG. 4B, in the case of Glycyrrhiza uralensis-derived exosomes (Embodiment), it may be confirmed that it shows superior cell proliferation effect than Glycyrrhiza uralensis extract-derived exosomes (Comparative Example 3) from 0.031% to 0.5%, and shows the most excellent effect at 0.125%.

### Experimental Example 5

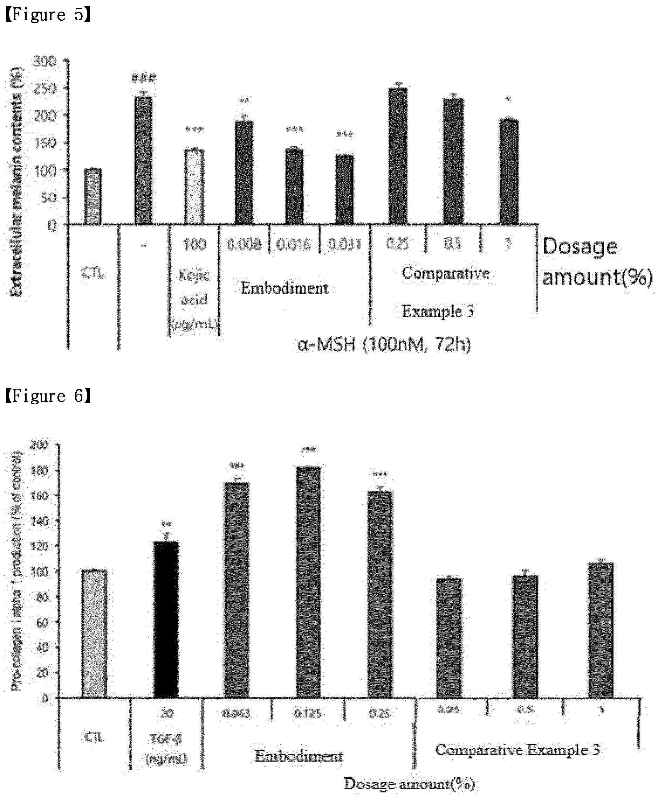

In order to confirm the whitening effect of the exosomes of the Embodiment and Comparative Example 3, the following experiment was conducted, and the results are shown in FIG. 5.

Melanin production was measured using a modified version of the method by Hosoi et al. (1985). Cultured B16F10 cells were seeded at 1 × 10⁵ cells/well in a 24-well plate and cultured for 24 hours. Each well was treated with 1 µg/ml of α-melanocyte-stimulating hormone (α-MSH) to induce melanin production for 24 hours. The exosomes of the Embodiment were treated at concentrations of 0.008%, 0.016%, and 0.031% (2 × 10¹⁰ particles/ml (100% basis)), and the exosomes of Comparative Example 3 were treated at concentrations of 0.25%, 0.5%, and 1% (2 × 10¹⁰ particles/ml (100% basis)), and then cultured for 72 hours. The negative control was untreated, and the positive control was treated with 100 µg/ml kojic acid.

After 72 hours of culturing, each well was washed with PBS. After washing, 400 µl of 0.2N NaOH solution was added, and the mixture was lysed at 60°C for 1 hour, and then the absorbance was measured at 405 nm using a microplate reader to determine the amount of extracellular melanin.

FIG. 5 is a graph illustrating the amount of extracellular melanin.

Referring to FIG. 5, in in the case of Glycyrrhiza uralensis-derived exosomes (Embodiment), it may be confirmed that there is a whitening effect from 0.008%, and in the case of Glycyrrhiza uralensis extract-derived exosomes (Comparative Example 3), it may be confirmed that the whitening effect appears at a very high concentration of 1%.

### Experimental Example 6

In order to confirm the anti-wrinkle efficacy of the exosomes of the Embodiment and Comparative Example 3, an evaluation of procollagen type I production ability was conducted, and the results thereof are shown in FIG. 6.

Human skin fibroblasts HS68 cells were cultured in DMEM medium containing 10% fetal bovine serum (FBS), penicillin 7.5 mg/l, and streptomycin 7.5 mg/l at 37°C for 24 hours under 5% CO₂. After culturing, the medium was discarded, the cells were washed with 10% PBS, and fresh medium was treated with the exosomes of the Embodiment at concentrations of 0.063%, 0.125%, and 0.25% (2 × 10¹⁰ particles/ml (100% basis)) and Comparative Example 3 exosomes at concentrations of 0.25%, 0.5%, and 1% (2 x 10¹⁰ particles/ml (100% basis)), and then cultured for 24 hours. Thereafter, the culture solution was collected and the amount of collagen produced in the culture solution was measured using a procollagen type-IC peptide (PIP) ELISA kit (takara). At this time, the negative control was treated with PBS, and the positive control was treated with TGF-β 1 at 5 ng/ml.

Referring to FIG. 6, when Glycyrrhiza uralensis-derived exosomes (Embodiment) were treated, the amount of collagen production increased starting at 0.063%, while Glycyrrhiza uralensis extract-derived exosomes (Comparative Example 3) showed no increase in collagen production even at 1%.

### Experimental Example 7

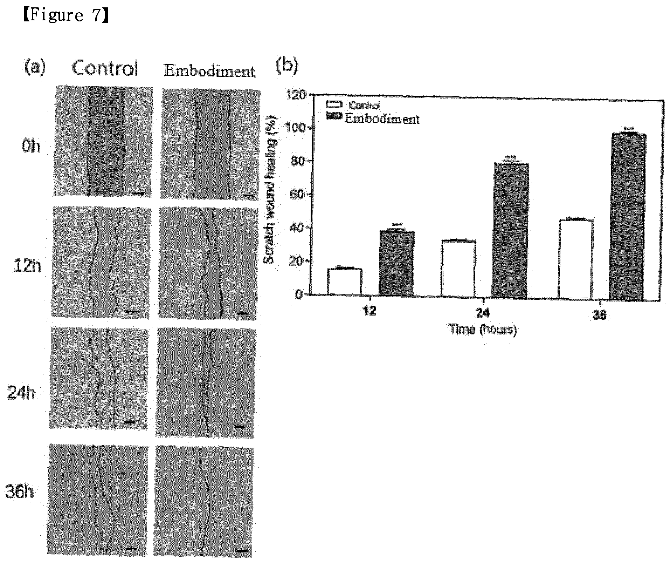

In order to confirm the wound healing efficacy of the exosomes extracted according to the Embodiment, wounds were inflicted on keratinocytes and then wound healing activity was confirmed, and the results thereof are shown in FIG. 7.

Specifically, 2.5×10⁵ cells/ml of human keratinocyte HaCaT cells were transferred to a 6-well plate and cultured for 24 hours in DMEM medium containing 10% (v/v) fetal bovine serum (FBS), and then a HaCaT cell monolayer was subjected to "scratch-damage" using a p200 pipette tip. The "scratch-damaged" HaCaT cell layer was treated with 1×10⁸ particles/ml of the exosomes extracted in the above Embodiment, and then further cultured for 12 hours, 24 hours, and 36 hours. Thereafter, the area ratio was calculated using the Scion-Image (Scion Corporation, MA) program.

FIG. 7A is a photograph morphologically comparing the scratched area and FIG. 7B is a graph showing the results of calculating the area ratio using the Scion-Image (Scion Corporation, MA) program. When the experimental group treated with the Embodiment was treated for 12 hours, 24 hours, and 36 hours, it may be confirmed that the "scratch damage" was healed and the scratched area was reduced, and it may be confirmed that the Glycyrrhiza uralensis-derived exosomes (Embodiment) had a significantly higher wound healing activity compared to the negative control.

As described above, the plant-derived exosome extraction method according to the present disclosure may improve the extraction efficiency of exosomes, and the cosmetic composition containing the extracted exosomes may exhibit excellent effects in anti-wrinkle, cell proliferation, wound healing, or whitening.

### INDUSTRIAL APPLICABILITY

The exosome isolation method according to the present disclosure can increase the isolation efficiency of exosomes and obtain highly stable exosomes, so it can be applied to cosmetics having anti-wrinkle, cell proliferation, wound healing, or whitening effects.

## Claims

1. A method for extracting plant-derived exosomes, the method comprising:
(a) washing and juicing a plant to obtain a plant juice;
(b) firstly centrifuging the plant juice to obtain a first supernatant;
(c) secondly centrifuging the first supernatant to obtain a second supernatant;
(d) subjecting the second supernatant to tangential flow filtration (TFF) to obtain an isolated substance; and
(e) adding antibody-attached beads to the isolated substance to obtain an extract containing plant-derived exosomes.

2. The method of claim 1, wherein in step (a), the plant is Glycyrrhiza uralensis.

3. The method of claim 1, wherein in step (b), the first centrifugation is performed at 1,000 xg to 3,000 xg for 10 to 50 minutes.

4. The method of claim 1, wherein in step (c), the second centrifugation is performed at 8,000 xg to 12,000 xg for 40 to 80 minutes.

5. The method of claim 1, wherein in step (d), the tangential flow filtration is performed using a TFF filter with a molecular weight cutoff (MWCO) of 50,000 Da to 200,000 Da.

6. The method of claim 1, wherein in step (e), the antibody is a plant-derived tetraspanin antibody.

7. A plant-derived exosome extracted by the method according to any one of claims 1 to 6.

8. A cosmetic composition comprising 0.1 to 20 wt% of the plant-derived exosomes of claim 7, based on the total weight of the composition.

9. The cosmetic composition of claim 8, wherein the cosmetic composition is for anti-wrinkle, cell proliferation, wound healing, or whitening purposes.
